(19) Europäisches Patentamt
European Patent Office
Office européen des brevets

(11) Publication number: 0 364 141 A1

# (12) EUROPEAN PATENT APPLICATION

(21) Application number: 89310040.4

(51) Int. Cl.5: C07D 413/12 , A01N 47/36

(22) Date of filing: 02.10.89

(30) Priority: 12.10.88 NZ 228212
03.04.89 AU 3508/89

(43) Date of publication of application:
18.04.90 Bulletin 90/16

(84) Designated Contracting States:
AT BE CH DE ES FR GB GR IT LI LU NL SE

(71) Applicant: ICI AUSTRALIA LIMITED
1 Nicholson Street
Melbourne Victoria 3001(AU)

(72) Inventor: Watson, Keith Geoffrey
18 Hamilton Avenue
Blackburn, VIC, 3130(AU)
Inventor: Drygala, Peter
22 The Avenue
Niddrie, VIC, 3042(AU)
Inventor: Bell, Stephen
18 Mirams Street
Ascot Vale, VIC, 3032(AU)

(74) Representative: Downes, John Edward et al
Imperial Chemical Industries PLC Legal
Department: Patents Po Box 6
Welwyn Garden City Herts, AL7 1HD(GB)

## (54) Herbicidal sulfonylurea derivatives.

(57) Compounds of the formula

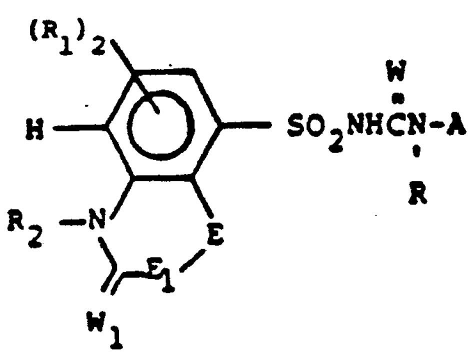

and salts thereof, W and W, being independently O and S, A being a nitrogen-containing heterocyclic ring system, E being O, S(O)m or $NR_3$ where m is 0-2, $R_1$, $R_2$ and $R_3$ is hydrogen, $C_1$-$C_4$ alkyl or $C_2$-$C_4$ alkenyl or alkynyl and $E_1$ being hydrogen, halogen or one of a variety of organic substituents.
The compounds are effective herbicides.

EP 0 364 141 A1

# HERBICIDAL SULFONYLUREA DERIVATIVES

This invention relates to organic compounds having biological activity and in particular to organic compounds having herbicidal properties and plant growth regulating properties, to processes for the preparation of such compounds, to intermediates useful in the preparation of such compounds and to herbicidal compositions and processes utilizing such compounds.

The use of certain sulfonylurea derivatives as herbicides is known in the art. Thus, for example, the "Pesticide Manual" (C R Worthing Editor, The British Crop Protection Council, 7th Edition 1983) describes the sulfonylurea derivative known commercially as chlorsulfuron [1-(2-chlorophenylsulfonyl)-3-(4-methoxy-6-methyl-1,3,5-triazin-2-yl) urea] and its use as a broadleaf weed herbicide in cereals. This compound is described in Australian Patent No. 510,056 and its equivalents such as U.S. Patent 4,127,405.

Australian Patent Application No. 89,354/82 (published April 21, 1983) discloses herbicidal benzenesulfonylureas including those of general formulae 1 and 2 :

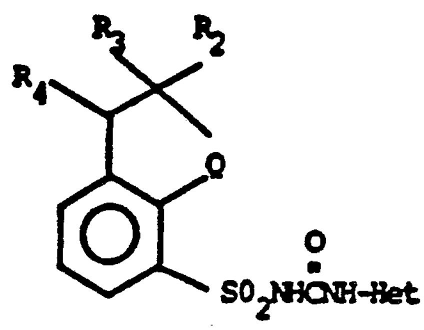

1

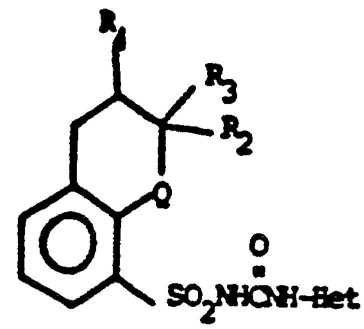

2

wherein
Q is O, S or $SO_2$;
$R_2$ is H or $C_1$ to $C_3$ alkyl;
$R_3$ and $R_4$ are H or $CH_3$;
Het is a pyrimidyl or triazinyl heterocyclic ring.

European Patent Application 82,681 (published June 29, 1983) discloses herbicidal benzene-sulfonylureas including those of general formulae 3 and 4:

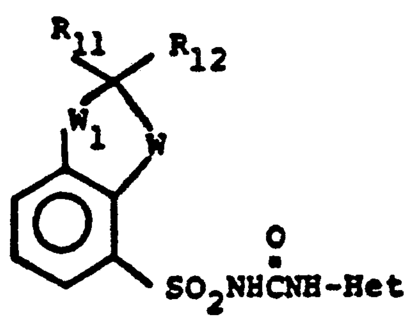

3

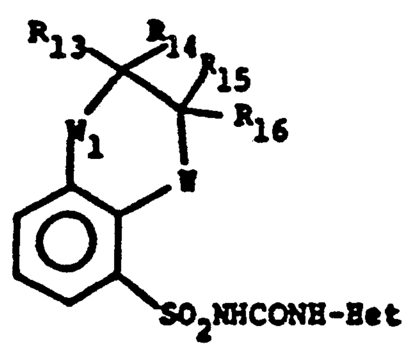

4

Wherein:
W and $W_1$ are O, S, SO or $SO_2$;
$R_{11}$ is H or $CH_3$;
$R_{12}$ is H or $C_1$ to $C_4$ alkyl;
$R_{13}$, $R_{14}$, $R_{15}$ and $R_{16}$ are independently H or $CH_3$;

Het is a pyrimidyl or triazinyl heterocyclic ring.

Australian Patent Application No. 20659/83 (published May 3, 1984) discloses herbicidal sulfonyl ureas including those of general formulae 5 and 6:

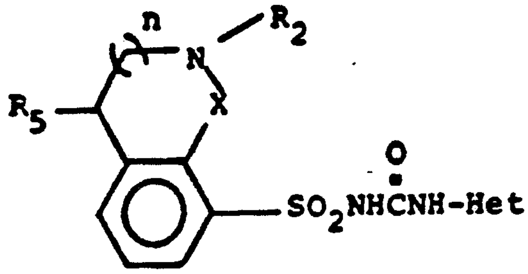

5

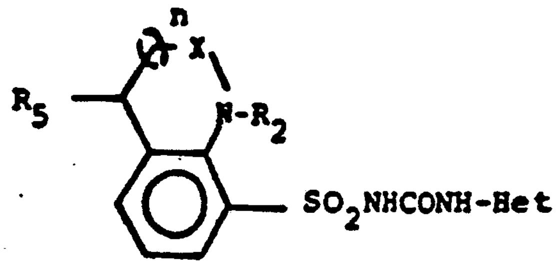

6

wherein

X is C=O or $SO_2$;

$R_2$ is H or $C_1$ to $C_4$ alkyl;

$R_5$ is H or $CH_3$;

n is 0, 1 or 2.

Australian Patent Application No. 16889/83 (published January 19, 1984) discloses herbicidal sulfonylureas of the general structure 7:

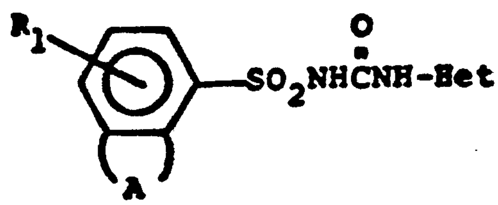

7

wherein

A is an unsubstituted or substituted bridge of 3 or 4 atoms which contains 1 or 2 oxygen, sulfur or nitrogen atoms and, together with the linking carbon atom, forms a non-aromatic 5- or 6-membered heterocyclic ring system, with the proviso that two oxygen atoms are separated by at least one carbon atom and that oxygen and sulfur atoms are only linked to each other if the sulfur atom takes the form of the -SO- or $SO_2$- group;

$R_1$ is hydrogen, halogen, $NO_2$, $C_1$-$C_4$ alkyl, $C_1$-$C_4$ haloalkyl, $C_1$-$C_4$ alkoxy, $C_1$-$C_4$ haloalkoxy, $C_1$-$C_4$ alkoxycarbonyl, $C_1$-$C_4$ alkylthio, $C_1$-$C_4$ alkylsulfinyl, $C_1$-$C_4$ alkylsulfonyl or $C_2$-$C_5$ alkoxyalkoxy; Het is a pyrimidyl or triazinyl heterocyclic ring.

Australian Patent Application No. 54,625/86 (published October 16, 1986) discloses herbicidal sulfonylureas including those of the general structure 8:

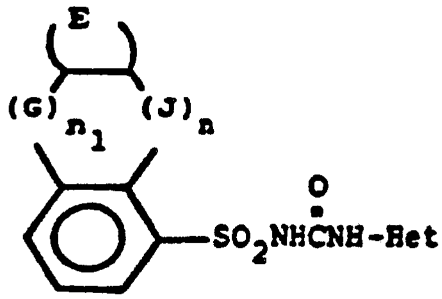

8

wherein

G is $CH_2$, $CH_2CH_2$, O, S, NH, $NCH_3$ or CH = CH;

J is $CH_2$, C = O, $(S(O)_m$, O, NH, $NCH_3$, CHOH, $CHOCH_3$, $CH(CH_3)$ or $C(CH_3)OH$; n and $n_1$ are independently 0 or 1, m is 0, 1 or 2; E is a bridge of 3 or 4 atoms containing 0 to 2 heteroatoms; Het is a pyrimidyl or triazinyl heterocyclic ring.

## Summary of the Invention

It has now been found that a new group of bicyclic sulfonylurea derivatives exhibit particularly useful herbicidal activity.

Accordingly the invention provides a compound of the formula I

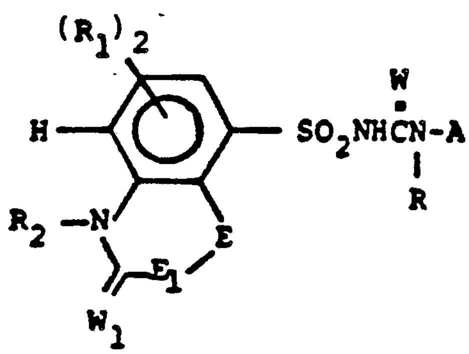

I

wherein

W and $W_1$ are independently O or S;

E is O, $S(O)_m$, or N-$R_3$;

$E_1$ is $CH_2$, $CH_2CH_2$, CH($C_1$-$C_4$ alkyl), $C(CH_3)_2$ or CH aryl; $R_1$, which may be the same or different, are halogen, $C_1$-$C_4$ alkyl, $C_1$-$C_4$ haloalkyl, nitro, $C_1$-$C_4$ alkoxy, $C_1$-$C_4$ haloalkoxy, cyano, $C_1$-$C_4$ alkoxycarbonyl, $C_1$-$C_4$ alkylthio, $C_1$-$C_4$ alkylsulfinyl, $C_1$-$C_4$ alkylsulfony, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino;

R, $R_2$ and $R_3$ are each independently hydrogen, $C_1$-$C_4$ alkyl, $C_2$-$C_4$ alkenyl, or $C_2$-$C_4$ alkynyl;

m = 0, 1 or 2

A is 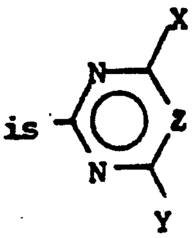 , 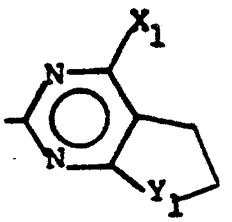 , 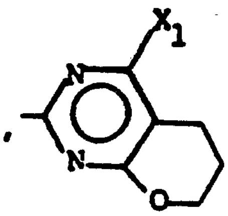 , 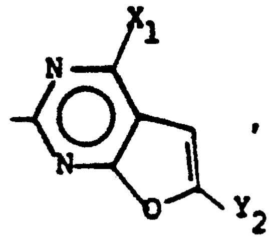 ,

A-1 A-2 A-3 A-4

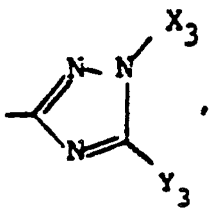 , 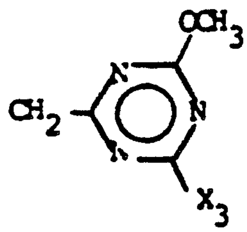 or 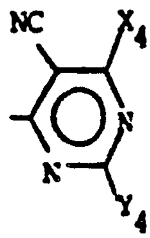

A-5 A-6 A-7

X is $CH_3$, $OCH_3$, $OCH_2CH_3$, Cl, F, Br, I, $OCF_2H$, $CH_2F$ or $CF_3$,
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH)_3CH_3$, $OCH_2CH$-$CH_2$, $OCH_2C$-$CH$, $OCH_2CF_3$, cyclopropyl, $OCH_2CH_2OCH_3$, $CH_2SCH_3$, $(C=O)R_4$, $CR_4(QCH_3)_2$,

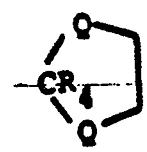 ,

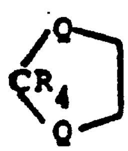 , 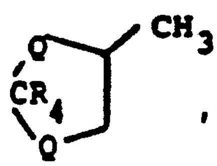 ,

$CR_4(OCH_2CH_3)_2$, $OCF_2H$, $SCF_2H$, C = CH or C = $CCH_3$ where Q is O or S and $R_4$ is H or $CH_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$Y_2$ is H or $CH_3$;
$X_2$ is $CH_3$, $C_2H_5$ or $CH_2CF_3$;
$Y_3$ is $OCH_3$, $OC_2H_5$, $SCH_3$, $SC_2H_5$, $CH_3$ or $CH_2CH_3$;
$X_3$ is $CH_3$ or $OCH_3$;
$Y_4$ is $CH_3$, $OCH_3$, $OCH_2CH_3$ or Cl; and
$X_4$ is $CH_3$, $OCH_3$, $OCH_2CH_3$, $CH_2OCH_3$ or Cl.

The invention also comprises the salts which the compounds of formula 1 are able to form with amines, alkali metal bases and alkaline earth metal bases, or with quaternary ammonium bases.

In the above definitions, the term "alkyl" used either alone or in compound words such as "alkylthio" or "haloalkyl", denotes straight chain or branched alkyl, e.g., methyl, ethyl, n-propyl, isopropyl or the different butyl isomers.

Alkoxy denotes methoxy, ethoxy, n-propoxy, isopropoxy and the different butyl isomers.

Alkenyl denotes straight chain or branched alkenes, e.g., vinyl, 1-propenyl, 2-propenyl, 3-propenyl or

the different butenyl isomers.

Alkylsulfonyl denotes methylsulfonyl, ethylsulfonyl or the different propylsulfonyl isomers.

Alkylthio, alkylsulfinyl, alkylamino, etc. are defined in an analogous manner.

Preferred values of E, are $CH_2$, $CH_2CH_2$, $CH(C_1\text{-}C_4\ alkyl)$ and $C(CH_3)_2$.

Preferred compounds of the invention include:

1) Those compounds of formula I where W is O and A is A-1.

2) Compounds of Preferred 1) where R is hydrogen;

$R_1$ is fluorine, chlorine, bromine, methyl, methoxy, trifluoromethyl or methylthio; and

Y is methyl, methoxy, ethoxy, methoxymethyl, trifluoromethyl, 2,2,2-trifluoroethoxy, dimethoxymethyl or difluoromethoxy.

A further group of preferred compounds of formula I comprises those compounds wherein :

E is O, S, NH or $NCH_3$;

$E_1$ is $CH_2$, $CH_2CH_2$, or $CH(CH_3)$;

$W_1$ is O, and $R_2$ is hydrogen, methyl or ethyl.

Examples of the basic types of condensed saturated heterocyclic systems which are attached to the sulfonyl end of the sulfonylurea bridge are:

3-oxo-3,4-dihydro-2H-1,4-benzoxazines $B_1$,
3-oxo-3,4-dihydro-2H-1,4-benzothiazines $B_2$,
2-oxo-1,2,3,4-tetrahydroquinoxalines $B_3$,
2,3-dihydro-1,5-benzoxazepin-4-ones $B_4$,
2,3-dihydro-1,5-benzothiazepin-4-ones $B_5$, and
3,4-dihydro-1,5-benzodiazepine-2-ones $B_6$.

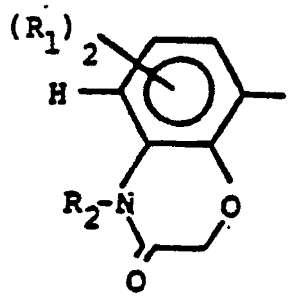

$B_1$

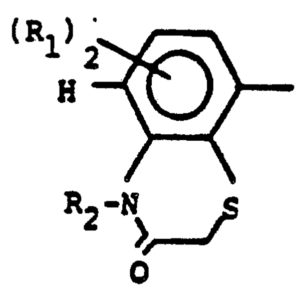

$B_2$

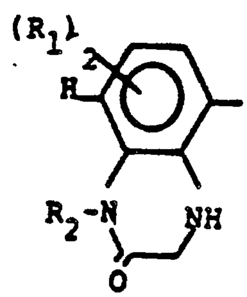

$B_3$

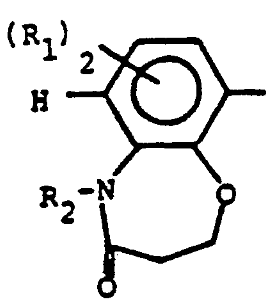

$B_4$

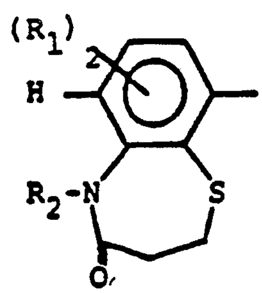

$B_5$

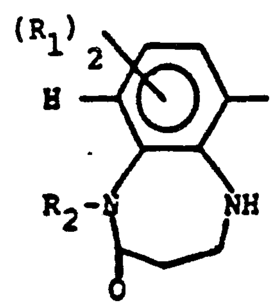

$B_6$

Especially preferred compounds are those of the formula:

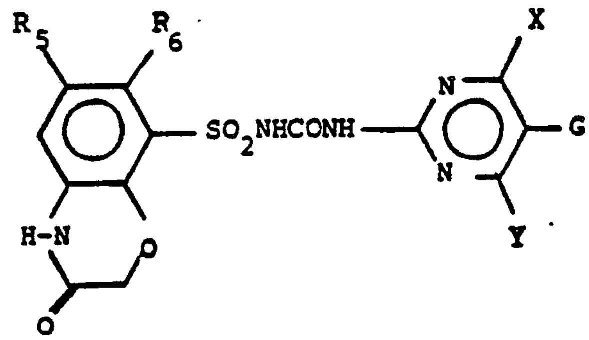

wherein $R_5$ and $R_6$ are selected from one of the following combinations;

(a) both are chloro;

(b) both are methyl;

(c) both are methoxy; and

(d) $R_5$ is chloro and $R_6$ is methoxy;

and X, G, and Y are selected as follows;

(e) when the groups $R_5$, $R_6$ are both chloro or both methoxy, X and Y are both methoxy and G is hydrogen;

(f) when the groups $R_5$, $R_6$ are both methyl, X, G and Y are selected from one of the following combinations;

(i) X and Y are both methoxy and G is hydrogen; and

(ii) X is methyl and G and Y together from a chain of three methylene groups which link the carbon atoms on the pyrimidine ring to which G and Y are attached; and

(g) when $R_5$ is chloro and $R_6$ is methoxy, X, G and Y are selected from one of the following combinations;

(i) X is methyl, G is hydrogen and Y is methoxy; and

(ii) X and Y are both methoxy and G is hydrogen

Specific examples of the compounds of the invention include those compounds listed in Tables Ia - Ib.

Table 1a

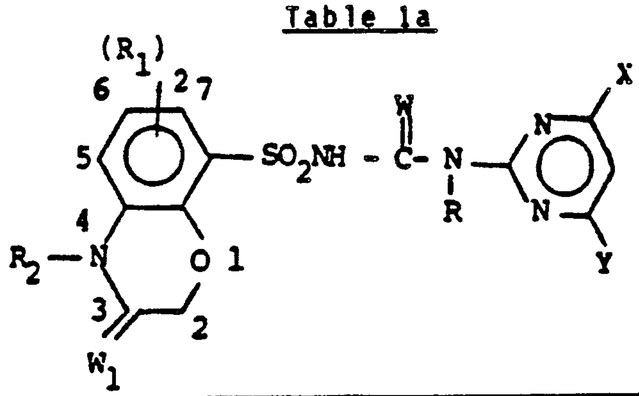

| Compound No. | $R_1$ | $R_2$ | $W_1$ | W | R | X | Y |
|---|---|---|---|---|---|---|---|
| 1 | $6,7-Cl_2$ | H | O | O | H | OMe | OMe |
| 2 | $6,7-Me_2$ | H | O | O | H | OMe | OMe |
| 3 | $6,7-(OMe)_2$ | H | O | O | H | OMe | OMe |
| 4 | 6-Cl,7-OMe | H | O | O | H | Me | OMe |
| 5 | 6-Cl,7-OMe | H | O | O | H | OMe | OMe |
| 7 | 6-Br,7-Me | H | O | O | H | OMe | OMe |
| 8 | 6-Br,7-Me | Me | O | O | H | OMe | OMe |

Table Ib

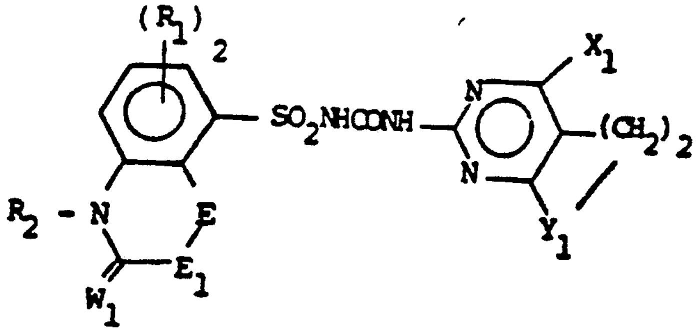

| Compound No. | $R_1$ | $R_2$ | $W_1$ | $E_1$ | E | $X_1$ | $Y_1$ |
|---|---|---|---|---|---|---|---|
| 6 | 6,7-$Me_2$ | H | O | $CH_2$ | O | Me | $CH_2$ |

The compounds of the invention may be prepared by a variety of methods and in a further aspect the invention provides methods for the preparation of compounds of formula I.

Conveniently the preparation of the compounds of the invention can be considered in three parts.

Part A involves the preparation of fused phenylsulfonamides of the formula II

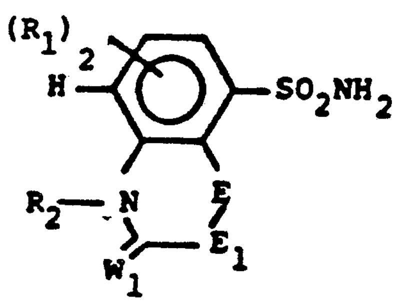

II

wherein $R_1$, $R_2$, $W_1$, E and $E_1$ are as defined for formula I. The sulfonamides of formula II can be prepared in a variety of ways including the general methods outlined below.

(a) The preparation of aromatic sulfonamides from the corresponding anilines by diazotization and reaction of the diazonium salt with sulfur dioxide is well known in the art (J. Org; Chem;. 25, 1824 (1960).

$$ArNH_2 \xrightarrow[\text{2) } SO_2,\ CuCl_2]{\text{1) } NaNO_2/HCl} ArSO_2Cl \xrightarrow{NH_3} ArSO_2NH_2$$

Some of the necessary amino-benzo-azinones III have been described in the literature (see for example Rodds Chemistry of carbon compounds, Vol. IV H, p 463, 1978) and they may be prepared for example by

reduction of the corresponding nitro compounds.

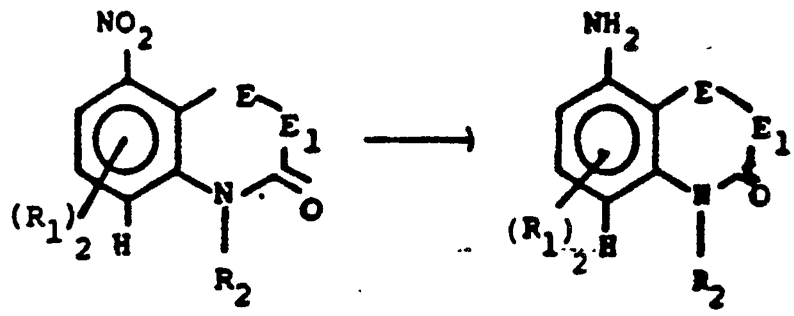

IIIa IIIb

Alternatively the amino compounds IIIb ($R_2$ = H) may be prepared by cyclization of certain 1,3-diamino benzenes IV which in turn are generally

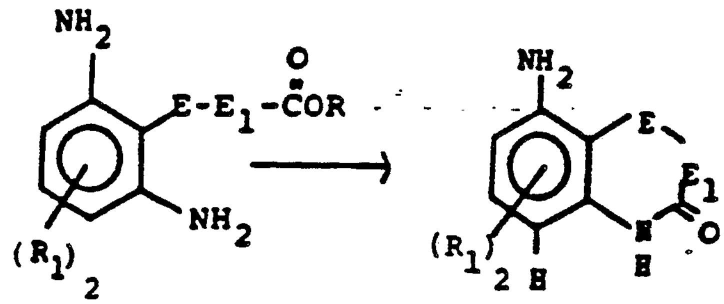

IV IIIb ($R_2$=H)

accessible from the corresponding dinitro benzenes V.

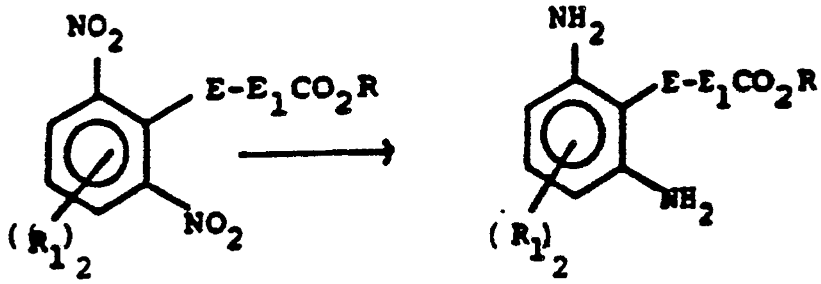

V IV

b) The bicyclic aromatic sulfonamides II may also be prepared from suitable 3-nitro benzene sulfonamides VI which have a leaving group X (preferably halogen) at the 2 position.

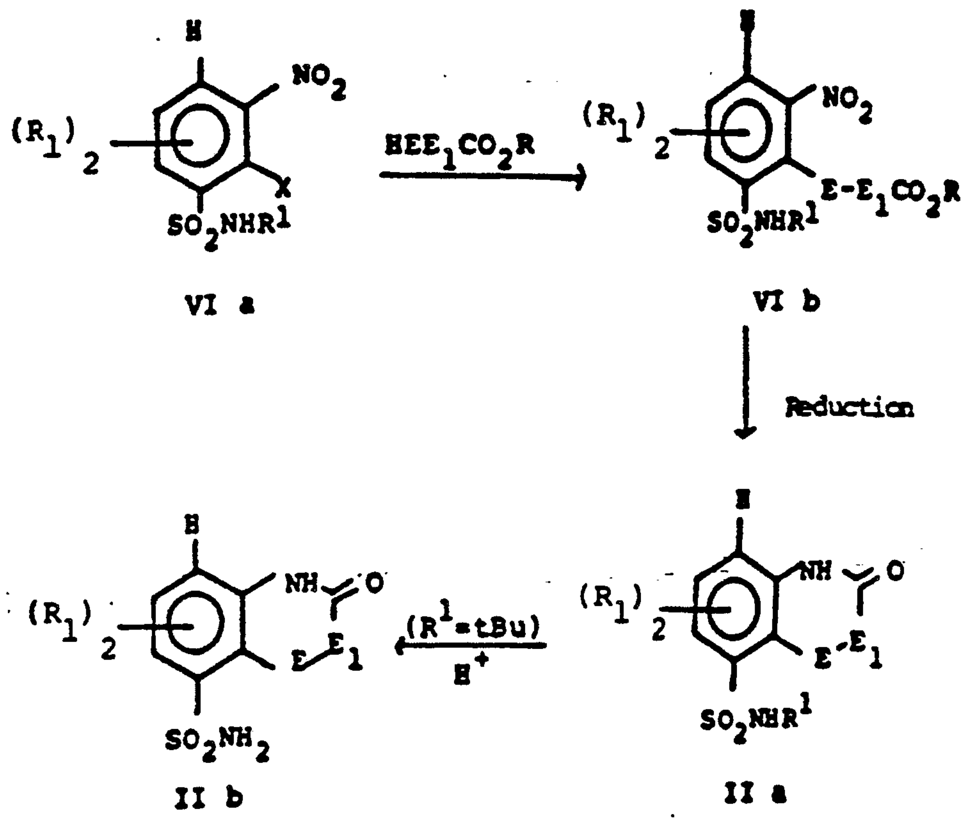


VI a

VI b

II b

II a

c) Another route to the sulfonamides II (E = O) involves

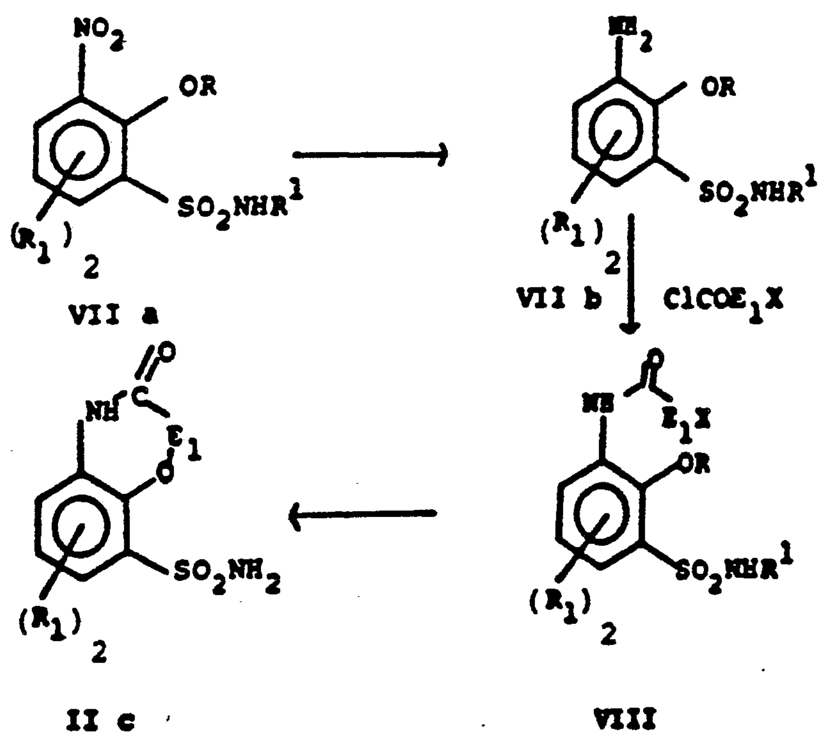


VII a

VII b

II c

VIII

use of 3-nitro-2-alkoxybenzenesulfonamides VII. Reduction of the nitro group followed by acylation of the amino group gives acylamino benzene-sulfonamides VIII. In the case where R is hydrogen the heterocyclic ring system is readily formed by treatment of the compound VIII with a base. If R is an alkyl group in compound VIII then dealkylation with for example aluminium chloride is carried out before the cyclisation

step.

d) Preparation of compounds of formula II in which $R_2$ is not hydrogen can be carried out by alkylation of the nitrogen using standard techniques.

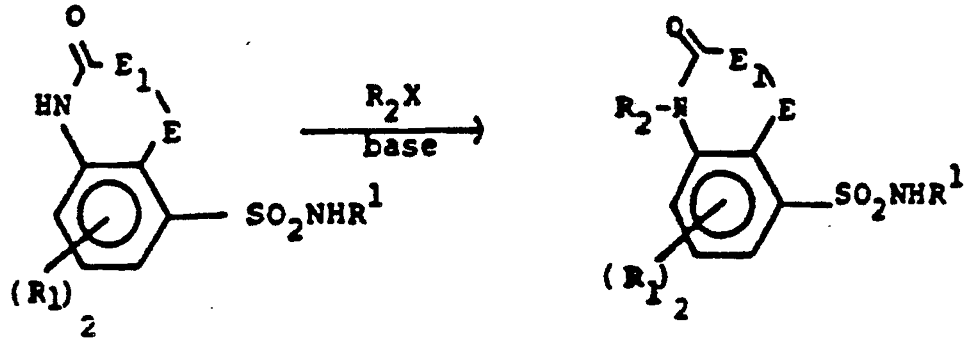


II a II d

e) Compounds of formula II in which $W_1$ is sulfur may be prepared from the corresponding oxygen compounds by treatment with reagents such as phosphorous pentasulfide and Lawessons reagent.

In each of parts a), b), c) and d) above group R may be either hydrogen or an alkyl group and group $R^1$ is either hydrogen or a tertiary butyl group.

In parts b), c) and d) substituent X is a leaving group preferably a halogen atom.

Part B of the preparation of the compounds of the invention involves the preparation of the various nitrogen-containing heterocycles A-1 to A-7.

The heterocyclic amines of Formula IX can be prepared by methods known in the literature, or simple modifications thereof, by one skilled in the art.

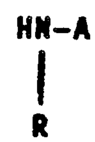

IX

For a review of the synthesis and reactions of 2- amino- and 2-methylaminopyrimidines (IX, A-A-1, Z = CH) see The Chemistry of Heterocyclic Compounds, Vol. 16, Wiley-Interscience, New York (1962). For a review of the synthesis and reactions of 2-amino- and 2-methylamino-s-triazines (IX, A=A-1, Z=N) see The Chemistry of Heterocyclic Compounds, Vol. 13, Wiley-Interscience, New York (1959), and F. C. Schaefer and K. R. Huffman, J. Org. Chem., 28, 1812 (1963). EP-A No. 84,224 and W. Braker et al., J. Amer. Chem. Soc., 69, 3072 (1947) describes methods for preparing aminopyrimidines and triazines substituted by acetal groups such as dialkoxymethyl or 1,3-dioxolan-2-yl. U.S. Patent 4,515,626 describes methods for the synthesis of cyclopropyl-pyrimidines and triazines substituted by such groups as alkyl, haloalkyl, alkoxy, haloalkoxy, alkylamino, dialkylamino or alkoxyalkyl.

The 5,6-dihydrofuro[2.3-d]pyrimidine-2-amines, the cyclopenta[d]pyrimidines-2-amines (IX, A is A-2) and the 6,7-dihydro-5H-pyrano[2,3-d]pyrimidin-2-amines (IX, A is A-3) can be prepared as taught in U.S. 4,339,267. The furo[2,3-d]pyrimidin-2-amines (IX, A is A-4) are described in U.S. 4,487,626.

Compounds of formula IX where A is A-5, are described in EP-A-73,562. Compounds of Formula I where A is A-6, are described in U.S. 4,496,392.

The amines of formula IX where A is A-7 can be prepared by methods taught in European Publication No. 125,864 (published 21/11/84) or by suitable modifications that would be obvious to one skilled in the art.

Part C of the preparation of the compounds of the invention (formula I) involves the coupling of the sulfonamides of formula II with the heterocyclic amines of formula IX. The compounds of formula I can be prepared by one or more of the methods described below.

a) Many of the compounds of formula I can be prepared by reacting a sulfonylisocyanate or a sulfonylisothiocyanate of formula X with a heterocyclic amine of formula IX.

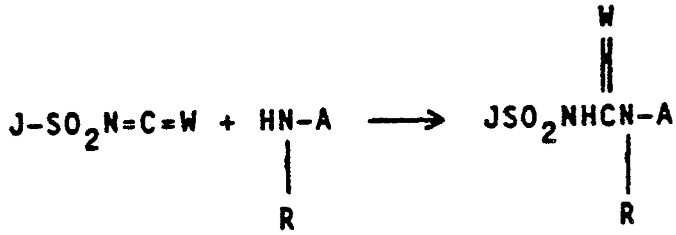

X IX I

where J represents the bicyclic system

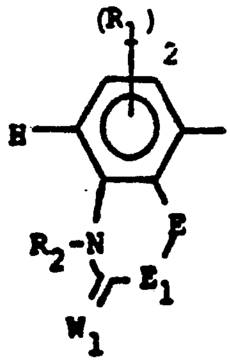

and W, A and R are as previously defined.

The reaction is carried out at 25° to 100°C in an inert, aprotic solvent such as methylene chloride or xylene for 0.5 to 24 hours as taught in U.S. Patent 4,127,405

The intermediate sulfonylisocyanates (X, W=O) and isothiocyanates (X, W=S) are prepared by a variety of methods which are well known in the art and are described for example in European Patent Application 212,779 and the references cited therein.

b) Many of the compounds of formula I, where W is oxygen, can be prepared by reacting a phenyl carbamate of formula X1 with a suitable amine of formula IX.

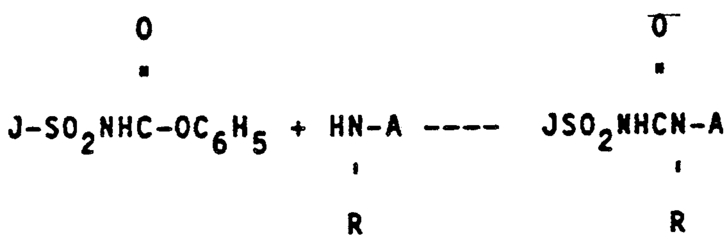

XI IX Ia

The reaction is carried out at 50° to 100°C in a solvent such as dioxane for 0.5 to 24 hours. The required carbamates XI are prepared by reacting the corresponding sulfonamides II with diphenylcarbonate in the presence of a strong base.

c) Compounds of formula Ia can also be made by reacting a heterocyclic carbamate of formula XII with a suitable sulfonamide of formula II.

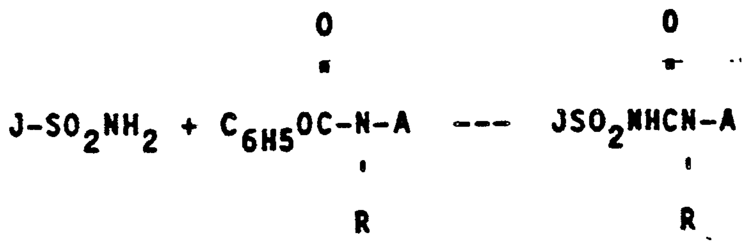

II XII Ia

The reaction is carried out at 0° to 100°C in a solvent such as acetonitrile or dioxane in the presence of a non-nucleophilic base such as DBU for 0.2 to 24 hours. The required phenylcarbamates XII are prepared by reacting the corresponding heterocyclic amines IX with diphenylcarbonate or phenylchloroformate in the presence of a strong base.

d) Some of the compounds of the invention of formula Ib can be prepared by reacting a sulfonamide II with a heterocyclic isothiocyanate of formula XIII.

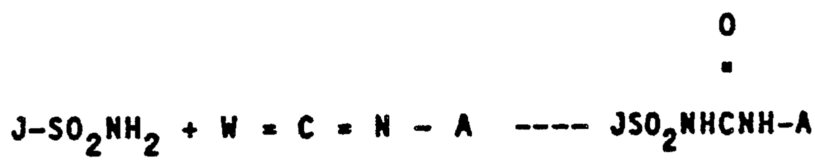

II XIII Ib

The reaction is carried out at 25° to 80°C in an inert, aprotic solvent such as acetone or acetonitrile in the presence of a base such as potassium carbonate for 0.5 to 24 hours. The required heterocyclic isocyanates and iso-thiocyanates XIII are prepared from the corresponding amines $H_2NA$ which would be known to one skilled in the art as taught in EPO Publication 35,893.

In each of parts b), c) and d) above the groups J, W, A and R are as previously described.

Certain of the intermediate compounds of formulae II, III VI, VII and VIII are novel compounds and therefore in further embodiments the invention provides novel compounds of formulae II, III, VI, VII and VIII and processes for the preparation thereof.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g. hydroxide, alkoxide, carbonate or hydroxide). Quaternary amine salts can be made by similar techniques.

Salts of compounds of Formula I can also be prepared by exchange of one cation to another. Cationic exchange can be effected by direct treatment of an aqueous solution of a salt of a compound of Formula I, (e.g, alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I, (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I, with a suitable acid, e.g., p-toluenesulfonic acid, trichloroacetic acid or the like.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

The compounds of formula I may be applied directly to the plant (post-emergence application) or to the

soil before the emergence of the plant (pre-emergence application).

Generally speaking the compounds of the present invention are highly active pre-emergent and especially post-emergent herbicides. Some of them have utility for broad spectrum total vegetation control. Other compounds of the invention have utility for selective weed control in crops such as sugar beet, soya bean, cotton and rice.

Accordingly, in yet a further aspect the invention provides a process for controlling weeds in cultivated crops which process comprises applying to the crop, or to the growth medium of the crop, a compound of formula I, as hereinbefore defined, in an amount sufficient to severely damage or kill the weeds but insufficient to damage the crop substantially.

Certain of the compounds of formula I exhibit useful plant growth regulating activity.

Plant growth regulating effects may be manifested in a number of ways. For example, suppression of apical dominance, stimulation of auxiliary bud growth, stimulation of early flowering and seed formation, enhancement of flowering and increase in seed yield, stem thickenings, stem shortening and tillering.

Accordingly in a still further aspect the invention provides a process for regulating the growth of a plant which process comprises applying to the plant, to the seed of the plant, or to the growth medium of the plant, an effective amount of a compound of formula I, as hereinbefore defined.

To effect the plant growth regulating process of the present invention the compounds of formula I may be applied directly to the plant (post-emergence application) or to the seed or soil before the emergence of the plant (pre-emergence) application.

The compounds of formula I may be used on their own to regulate the growth of plants but in general are preferably used in the form of a composition comprising a compound of the invention in admixture with a carrier comprising a solid or liquid diluent. Therefore, in a still further aspect the invention provides plant growth regulating compositions comprising a compound of formula I as hereinbefore defined and an inert carrier therefor.

The compositions of the present invention may be in the form of solids, liquids or pastes. The compositions include both dilute compositions which are ready for immediate use and concentrated compositions which may require dilution before use. Therefore, the concentration of the active ingredient in the compositions of the present invention will vary depending on the types of formulation and whether the composition is ready for use such as, for example, a dust formulation or an aqueous emulsion or whether the composition is a concentrate such as, for example, an emulsifiable concentrate or a wettable powder, which is suitable for dilution before use. In general, the composition of the present invention comprise from 1 ppm to 99% by weight of active ingredient.

The solid compositions may be in the form of powders, dusts, pellets, grains, and granules wherein the active ingredient is mixed with a solid diluent. Powders and dusts may be prepared by mixing or grinding the active ingredient with a solid carrier to give a finely divided composition. Granules, grains and pellets may be prepared by bonding the active ingredient to a solid carrier, for example, by coating or impregnating the preformed granular solid carrier with the active ingredient or by agglomeration techniques.

Examples of solid carriers include: mineral earths and clays such as, for example, kaolin, bentonite, kieselguhr, Fuller's earth, Attaclay, diatomaceous earth, bole, loess, talc, chalk, dolomite, limestone, lime, calcium carbonate, gypsum, calcium sulfate, pyrophyllite, silicic acid, silicates and silica gels, fertilizers such as, for example, ammonium sulfate, ammonium phosphate, ammonium nitrate and urea; natural products of vegetable origin such as, for example, grain meals and flours, bark meals, wood meals, nutshell meals and cellulosic powders; and synthetic polymeric materials such as, for example, ground or powdered plastics and resins.

Alternatively, the solid compositions may be in the form of dispersible or wettable dusts, powders, granules or grains wherein the active ingredient and the solid carrier are combined with one or more surface active agents which act as wetting, emulsifying and/or dispersing agents to facilitate the dispersion of the active ingredient in liquid.

Examples of surface active agents include those of the cationic, anionic and non-ionic type. Cationic surface active agents include quaternary ammonium compounds, for example, the long chain alkylammonium salts such as cetyltrimethylammonium bromide. Anionic surface active agents include: soaps or the alkali metal, alkaline earth metal and ammonium salts of fatty acids; the alkali metal, alkaline earth metal and ammonium salts of ligninsulfonic acid; the alkali metal, alkaline earth metal and ammonium salts of arylsulfonic acids including the salts of naphthalenesulfonic acids such as butylnaphthalenesulfonic acids, the di- and tri- isopropylnaphthalenesulfonic acids, the salts of the condensation products of sulfonated naphthalene and naphthalene derivatives with formaldehyde, the salts of the condensation products of sulfonated naphthalene and naphthalene derivatives with phenol and formaldehyde, and the salts of alkylarylbenzenesulfonic acids such as dodecylbenzenesulfonic acid; the alkali metal, alkaline earth metal

and ammonium salts of the long chain mono esters of sulfuric acid or alkylsulfates such as laurylsulfate and the mono esters of sulfuric acid with fatty alcohol glycol ethers. Nonionic surface active agents include: the condensation products of ethylene oxide with phenols and alkylphenols such as isooctylphenol, octylphenol and nonylphenol; the condensation products of ethylene oxide with castor oil; the partial esters derived from long chain fatty acids and hexitol anhydrides, for example sorbitan monolaurate, and their condensation products with ethylene oxide; ethylene oxide/propylene oxide block copolymers; lauryl alcohol polyglycol ether acetal; and the lecithins.

The liquid compositions may comprise a solution or dispersions of the active ingredient in a liquid carrier optionally containing one or more surface active agents which act as wetting, emulsifying and/or dispersing agents. Examples of liquid carriers include: water, mineral oil fractions such as, for example, kerosene, solvent naphtha, petroleum, coal tar oils and aromatic petroleum fractions; aliphatic, cycloaliphatic and aromatic hydrocarbons such as, for example, paraffin, cyclohexane, toluene, the xylenes, tetrahydronaphthalene and alkylated naphthalenes; alcohols such as, for example, methanol, ethanol, propanol, isopropanol, butanol, cyclohexanol and propylene glycol; ketones such as, for example, dimethylformamide, dimethylsulfoxide, N-methylpyrrolidone and sulfolane.

A preferred liquid composition comprises an aqueous suspension, dispersion or emulsion of the active ingredient which is suitable for application by spraying, atomizing or watering. Such aqueous compositions are generally prepared by mixing concentrated compositions with water. Suitable concentrated compositions include emulsion concentrates, pastes, oil dispersions, aqueous suspensions and wettable powders. The concentrates are usually required to withstand storage for prolonged periods and after such storage to be capable of dilution with water to form aqueous preparations which remain homogeneous for a sufficient time to enable them to be applied by conventional spray equipment. The concentrates conveniently contain from 10 to 99%, preferably 10 to 60%, by weight of active ingredient.

Emulsion or emulsifiable concentrates are conveniently prepared by dissolving the active ingredient in an organic solvent containing one or more surface active agents and optionally an oil. Oil dispersions may be prepared by grinding together the active ingredient, a hydrocarbon oil, and one or more surface active agents. Aqueous suspension concentrates may conveniently be prepared by ball milling a mixture of the active agent and preferably at least one suspending agent. Suitable suspending agents include: hydrophilic colloids such as, for example, poly(N-vinylpyrrolidone), sodium carboxymethylcellulose and the vegetable gums, gum acacia and gum tragacanth; hydrated colloidal mineral silicates such as, for example, montmorillonite, beidellite, nontronite, hectorite, saponite, sauconite and bentonite; other cellulose derivatives; and poly(vinyl alcohol). Wettable powder concentrates may conveniently be prepared by blending together the active ingredient, one or more surface active agents, one or more solid carriers and optionally one or more suspending agents and grinding the mixture to give a powder having the required particle size.

The aqueous suspensions, dispersions or emulsions may be prepared from the concentrated compositions by mixing the concentrated compositions with water optionally containing surface active agents and/or oils.

It should be noted that the compounds of the invention of formula I are acidic. Therefore, the compounds of formula I may be formulated and applied as the salts of organic or inorganic bases. In formulating and employing the compounds of formula I in the form of their salts either the salts per se may be used in the formulation or the compounds of formula I may be used in the formulation and the salts generated in situ by the use of the appropriate organic or inorganic base.

The mode of application of the compositions of the invention will depend to a large extent on the type of composition used and the facilities available for its application. Solid compositions may be applied by dusting or any other suitable means for broadcasting or spreading the solid. Liquid compositions may be applied by spraying, atomizing, watering, introduction into the irrigation water, or any other suitable means for broadcasting or spreading the liquid.

The rate or application of the compounds of the invention will depend on a number of factors including, for example, the compound chosen for use, the identify of the plants whose growth is to be inhibited, the formulations selected for use and whether the compound is to be applied for foliage or root uptake. As a general guide, however, an application rate of from 0.001 to 10 kilograms per hectare is suitable while from 0.01 to 5.0 kilogram per hectare may be preferred.

The composition of the invention may comprise, in addition to one or more compounds of the invention, one or more compounds not of the invention but which possess biological activity. As a result, in certain applications the herbicidal use of the compounds of the invention alone may not be sufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

The compounds of this invention and their preparation are further illustrated by the following examples.

Example 1

(i) 2-Amino-4,5-dimethyl phenol

Sodium dithionite (16 g) in water (50 ml) was added gradually with stirring to a solution of 4,5-dimethyl-2-nitrophenol (2.4 g) in hot water (25 ml) and ethanol (50 ml). After the addition, the solution was allowed to cool to room temperature and, on standing and evaporation of the ethanol, a colourless precipitate gradually formed. The precipitate was collected by filtration and after air drying gave 1.84 g of 2-amino-4,5-dimethylphenol.

(ii) 6,7-Dimethyl-2H-1,4-benzoxazin-3(4H)-one

To a solution of 2-amino-4,5-dimethylphenol (1.80 g) in isobutyl methyl ketone (20 ml) was added a solution of sodium hydrogen carbonate (3.0 g) in water (15 ml) and the resulting mixture was stirred at room temperature. Chloroacetyl chloride (1.7 g) was added dropwise to the vigorously stirred mixture and, once the addition was complete the mixture was heated under reflux for 4 hours. On cooling and standing, a fine precipitate formed which was removed by filtration to give the benzoxazinone as a colourless solid (2.0 g). Proton magnetic resonance spectrum ($d_6$ acetone/$d_6$ DMSO): 2.14 (6H, s); 4.42 (2H, s); 6.42 (1H, s); 10.15 (1H, bs).

(iii) 6,7-Dimethyl-8-sulfamoyl-2H-1,4-benzoxazin-3(4H)-one

Chlorosulfonic acid (20 ml) was added to a stirred suspension of 6,7-dimethyl-2H-1,4-benzoxazin-3(4H)-one (1.95 g) in chloroform (10 ml) at room temperature. The resultant dark solution was heated at 60-80° under reflux for 3 hours and then carefully poured on to crushed ice. The crude sulfonylchloride was rapidly collected by filtration and immediately treated with excess dilute aqueous ammonia. The desired sulfonamide (2.1 g) was isolated as a nearly colourless solid and was filtered off and air dried. Proton magnetic resonance spectrum ($d_6$ DMSO); 2.14 (s, 3H); 2.32 (s, 3H); 4.48 (s, 2H); 6.83 (s, 1H); 7.1 (bs, 2H); 10.7 (bs, 1H).

(iv) 6,7-Dimethyl-8-(phenoxycarbonylsulfamoyl)2H-1,4-benzoxazin-3(4H)-one

Sodium hydride (0.10 g) was added to a cooled solution of 6,7-dimethyl-8-sulfamoyl-2H-1,4-benzoxazin-3(4H)-one (0.80 g) in dimethylformamide (10 ml). After stirring the cooled (5°) solution for 0.5 hours, diphenyl-carbonate (0.8 g) was added and the mixture was stirred at room temperature for 16 hours. The dimethylformamide was removed on a rotary evaporator and the residue was partitioned between ethyl acetate (20 ml) and dilute aqueous citric acid (50 ml). A colourless precipitate which did not dissolve in either layer was removed by filtration to give the sulfonamido phenyl carbamate as a white solid (0.8 g). Infrared spectrum (Nujol mull): 1770 and 1700 $cm^{-1}$.

(v) N-[(4,6-Dimethoxypyrimidin-2-yl)aminocarbonyl] 6,7-dimethyl-2H-1,4-benzoxazin-3(4H)-one-8-sulfonamide (2)

A suspension of dry 6,7-dimethyl-8-(phenoxycarbonylsulfamoyl)-2H-1,4-benzoxazin-3(4)-one (0.53 g) and 2-amino-4,6-dimethoxypyrimidine (0.20 g) in toluene (40 ml) was heated under reflux for 3 hours. After cooling to room temperature, the suspension was filtered to give the sulfonylurea as a white powder (500 mg). Proton magnetic resonance spectrum ($d_6$ DMSO): 2.20 (s, 3H); 2.40 (s, 3H); 3.89 (s, 6H); 4.40 (s, 2H);

5.96 (s, 1H); 6.93 (s, 1H); 10.1 (bs, 1H); 10.7 (bs, 1H); 12.5 (bs, 1H).

Example 2

Compound No. 6 was prepared from the sulfonamido phenyl carbamate of Example 1, part (iv) and 6,7-dihydro-4-methyl-5H-cyclopentapyrimidin-2-amine following essentially the same reaction conditions described in Example 1, part (v). Proton magnetic resonance spectrum ($d_6$ DMSO): 2.0 (bm, 2H); 2.21 (s, 3H); 2.37 (s, 3H); 2.46 (s, 3H); 2.8 (bm, 4H); 4.36 (s, 2H); 6.93 (s, 1H); 10.3 (s, 1H); 10.8 (s, 1H); 13.1 (s, 1H).

Example 3

Compound No. 7 and 8 were prepared from the appropriate 8-sulfamoyl-1,4-benzoxazin-3-ones by reaction with N-phenoxycarbonyl-4,6-dimethoxy-2-aminopyrimidine in dimethylformamide in the presence of diazobicyclo[5.4.0]undec-7-ene. The compounds were characterized by their pmr spectra :
compound 7 ($d_6$ DMSO): 2.68 (s, 3H); 3.92 (s, 6H); 4.54 (s, 2H); 6.02 (s, 1H); 7.39 (s, 1H); 10.59 (s, 1H exch); 10.98 (s, 1H exch); 12.87 (s, 1H exch).
compound 8 ($d_6$ DMSO): 2.72 (s, 3H); 3.76 (s, 3H); 3.92 (s, 6H); 4.61 (s, 2H); 6.01 (s, 1H); 7.71 (s, 1H); 10.54 (s, 1H exch); 12.85 (s, 1H exch).

Example 4

This non-limiting Example illustrates the preparation of formulations of a compound of the invention.

a) Emulsifiable Concentrate

Compound No 2 was dissolved in toluene/DMSO containing 7% v/v "Teric" N13 and 3% v/v "Kemmat" SC15B to give an emulsifiable concentrate which was diluted with water to the required concentration to give an aqueous emulsion which was applied by spraying.

("Teric" is a Trade Mark and "Teric" N13, is a product of ethoxylation of nonylphenol; "Kemmat" is a Trade Mark and "Kemmat" SC15B is a formulation of calcium dodecylbenzenesulfonate.)

b) Emulsifiable Concentrate

Compound No 2 (10 parts by weight), "Teric" N13 (5 parts by weight) and "Kemmat" SC15B (5 parts by weight) were dissolved in "Solvesso" 150 (80 parts by weight) to give an emulsifiable concentrate which may be diluted with water to the required concentration to give an aqueous emulsion which may be applied by spraying.

Example 5

The post-emergent herbicidal activity of the compound of the invention formulated as described in Example 3 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glass house, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glass house and sprayed with the required quantity of a composition of the invention. After spraying the boxes were returned to the glass house for a further 3 weeks and the effect of treatment was visually assessed by comparison with the untreated controls. The results are presented below. The damage to plants is rated on a scale from 0 to 5 where 0 represents from 0 to 10% damage, 1 represents from 11 to 30% damage, 2 represents from 31 to 60% damage, 3 represents from 61 to 80%

damage, 4 represents from 81 to 99% damage and 5 represents 100% kill. A dash (-) means that no experiment was carried out.

Compoud No.2

Application Rate (kg/ha) 0.4

| Plant | Degree of Damage |
|---|---|
| Wheat | 3 |
| Wild Oats | 3 |
| Ryegrass | 0 |
| Japanese millet | 3 |
| Barley | 4 |
| Peas | 0 |
| Ipomea | 4 |
| Mustard | 2 |
| Sunflower | 3 |

Example 6

Compound 2 was formulated for test by mixing an appropriate amount with 5 ml of an emulsion prepared by diluting 160 ml of a solution containing 21.9 g per litre of "Span" 80 and 78.2 g per litre of "Tween" 20 in methylcyclohexanone to 500 ml with water. ("Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide). 5 ml emulsion containing the test compound was then diluted to 40 ml with water and sprayed on to young pot plants (post-emergence test) of the species named below at a rate of 0.125 K g/ha. Damage to test plants was assessed after 14 days on a scale of 0 to 5 wherein 0 is 0 to 20% damage and 5 is complete kill.

The degree of herbicidal damage was assessed by comparison with untreated control plants. The results are given below. A dash (-) means that no experiment was carried out.

| Plant | Degree of Damage |
|---|---|
| Sugar Beet | 2 |
| Oilseed Rape | 4 |
| Cotton | 0 |
| Soya Bean | 3 |
| Maize | 4 |
| Winter wheat | 4 |
| Rice | 2 |
| Bidens pilosa | 4 |
| Ipomea | 2 |
| Amaranthus retroflexus | 3 |
| Polygonum | 2 |
| Chenopodium album | 0 |
| Galium aparine | 2 |
| Xanthium spinosum | 3 |
| Abutilon theophrasti | 3 |
| Cassia obtusifolia | 2 |
| Avena fatua | 4 |
| Digitaria sanguinalis | 4 |
| Alopecurus myosuroides | 4 |
| Setaria viridis | 3 |
| Echinochloa crus-galli | 4 |
| Sorghum halepense | 2 |
| Agropyron repens | 4 |
| Cyperus rotundus | 3 |

## Claims

1. A compound of formula I and the salts thereof

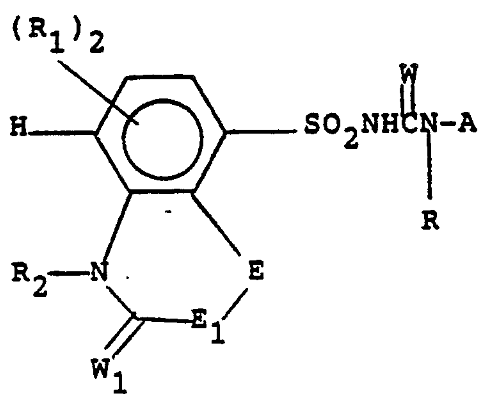

I

wherein
W and $W_1$ are independently O or S;
E is O, $S(O)_m$ or N-$R_3$;
$E_1$ is $CH_2$, $CH_2CH_2$, CH($C_1$-$C_4$ alkyl), $C(CH_3)_2$ or CH aryl; $R_1$ which may be the same or different halogen, $C_1$-$C_4$ alkyl, $C_1$-$C_4$ haloalkyl, $C_1$-$C_4$ alkoxy, $C_1$-$C_4$ halo-alkoxy, $C_1$-$C_4$ alkoxycarbonyl, $C_1$-$C_4$ alkylthio, $C_1$-$C_4$ alkylsulfinyl, $C_1$-$C_4$ alkylsulfony, sulfamoyl, $C_1$-$C_4$ alkylsulfamoyl, di($C_1$-$C_4$ alkyl)sulfamoyl, amino, $C_1$-$C_4$ alkylamino, or di($C_1$-$C_4$ alkyl) amino;
R, $R_2$ and $R_3$ are each independently hydrogen, $C_1$-$C_4$ alkyl, $C_2$-$C_4$ alkenyl, or $C_2$-$C_4$ alkynyl;
m = 0, 1 or 2;

A is 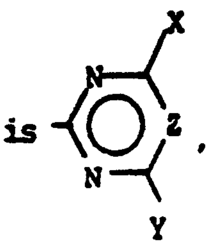, 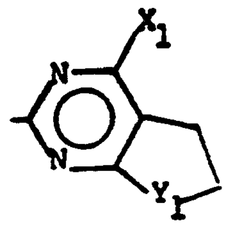, 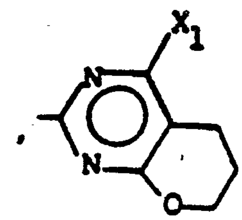, 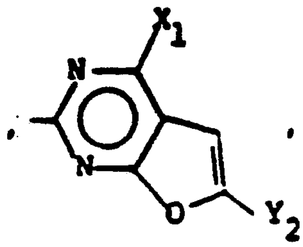,

A-1 A-2 A-3 A-4

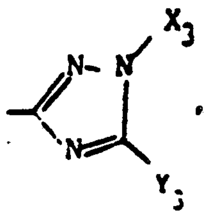, 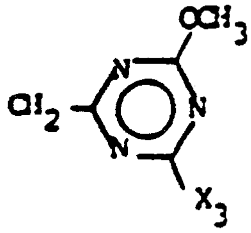 or 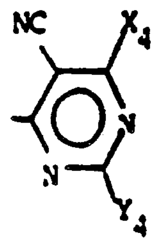

A-5 A-6 A-7

X is $CH_3$, $OCH_3$, $OCH_2CH_3$, Cl, F, Br, I, $OCF_2H$, $CH_2F$ or $CF_3$, Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH)_3CH_3$, $OCH_2CH\text{-}CH_2$, $OCH_2C\text{-}CH$, $OCH_2CF_3$, cyclopropyl, Q $OCH_2CH_2OCH_3$, $CH_2SCH_3$, $(C=O)R_4$, $CR_4(OCH_3)_2$,

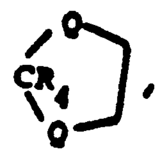,

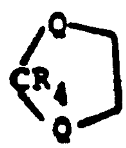, 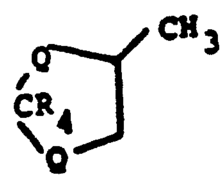,

$CR_4(OCH_2CH_3)_2$, $OCF_2H$, $SCF_2H$, C = CH or C = $CCH_3$ where Q is O or S and $R_4$ is H or $CH_3$;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$Y_2$ is H or $CH_3$;
$X_2$ is $CH_3$, $C_2H_5$ or $CH_2CF_3$;
$Y_3$ is $OCH_3$, $OC_2H_5$, $SCH_3$, $SC_2H_5$, $CH_3$ or $CH_2CH_3$;
$X_3$ is $CH_3$ or $OCH_3$;
$Y_4$ is $CH_3$, $OCH_3$, $OCH_2CH_3$ or Cl; and
$X_4$ is $CH_3$, $OCH_3$, $OCH_2CH_3$, $CH_2OCH_3$ or Cl.

2. A compound according to claim 1, wherein E, is selected from $CH_2$, $CH_2CH_2$, CH($C_1$-$C_4$ alkyl) or C-$(CH_3)_2$.

3. A compound according to claim 1, wherein W is O and A is A-1.

4. A compound according to claim 1, wherein
R is hydrogen; $R_1$ is fluorine, chlorine, bromine, methyl, methoxy, trifluoromethyl or methylthio; and
Y is methyl, methoxy, ethoxy, methoxymethyl, trifluoromethyl, 2,2,2-trifluoroethoxy, dimethoxymethyl or

difluoromethoxy.

5. A compound according to claim 1, wherein:
E is O, S, NH or $NCH_3$;
$E_1$ is $CH_2$, $CH_2CH_2$, or $CH(CH_3$;
$W_1$ is O, and $R_2$ is hydrogen, methyl or ethyl.

6. A compound of formula II

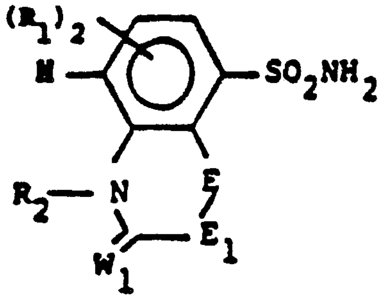

II

wherein $R_1$, $R_2$, $W_1$, E and $E_1$ are as defined in claim 1.

7. A compound of formula

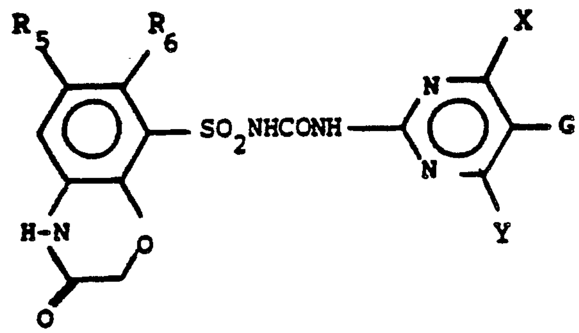

wherein the groups $R_5$, $R_6$ are selected from one of the following combinations;

(a) both are chloro;

(b) both are methyl;

(c) both are methoxy; and

(d) $R_5$ is chloro and $r_6$ is methoxy;

and D, G, and M are selected as follows;

(e) when the groups $R_5$, $R_6$ are both chloro or both methoxy, X and Y are both methoxy and G is hydrogen;

(f) when the groups $R_5$, $R_6$ are both methyl, X, G and Y are selected from one of the following combinations;

(i) X and Y are both methoxy and G is hydrogen; and

(ii) X is methyl and G and Y together form a chain of three methylene groups which link the carbon atoms on the pyrimidine ring to which G and Y are attached; and

(g) when $R_5$ is chloro and $R_6$ is methoxy, X, G and Y are selected from one of the following combinations;

(i) X is methyl, G is hydrogen and Y is methoxy; and

(ii) X and Y are both methoxy and G is hydrogen.

8. A compound selected from the group consisting of
N-[4,6-Dimethoxypyrimidin-2-yl)amino carbonyl]-6,7-dichloro-2H-1,4-benzoxazin-3(4H)-one-8-sulfonamide;
N-[(4,6-Dimethoxypyrimidin-2-yl)aminocarbonyl]-6,7 dimethoxy-2H-1,4-benzoxazin-3(4H)-one-8-sulfonamide;
N-[4,6-Dimethoxypyrimidin-2-yl)aminocarbonyl]6,7-dimethoxy-2H-1,4-benzoxazin-3(4H)-one-8-sulfonamide;
N-[(4-Methoxy-6-methylpyrimidin-2-yl)aminocarbonyl]-6-dichloro-7-methoxy-2H-1,4-benzoxazin-3(4H)-one-8-sulfonamide;
N-[(4,6-Dimethoxypyrimidin-2-yl)aminocarbonyl]-6-chloro-7-methoxy-2H-1,4-benzoxazin-3(4H)-one-8-sulfonamide; and
N-[6,7-Dihydro-4-methyl-5H-cyclopentapyrimidin-2-yl)amino carbonyl]-6,7-dimethyl-2H-1,4-benzoxazin-3-(4H)-one-8-sulfonamide.

9. A process for the preparation of a compound according to claim 1 by the reaction of a sulfonylisocyanate or a sulfonylisothiocyanate of formula X with a heterocyclic amine of formula IX.

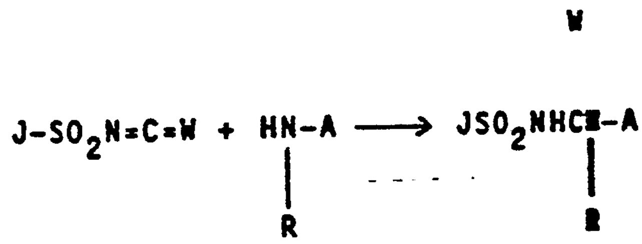

X IX I

where J represents the bicyclic system

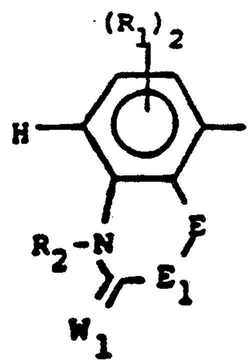

and W, A and R are as defined in claim 1.

10. A process for the preparation of a compound azccording to claim 1, where W is oxygen, by the reaction of a phenyl carbamate of formula XI with an amine of formula IX.

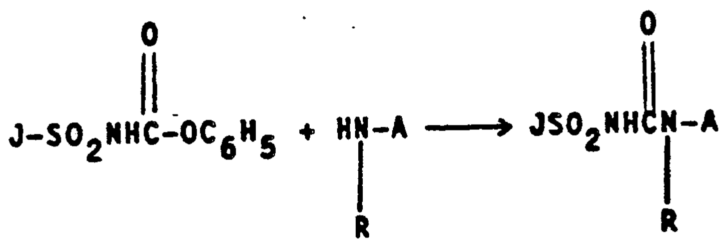

XI IX I

wherein A and J are as defined in claim 7.

11. A process for the preparation of a compound azccording to claim 1, where W is oxygen, by the reaction of a heterocyclic carbamate of formula XII with a sulfonamide of formula II.

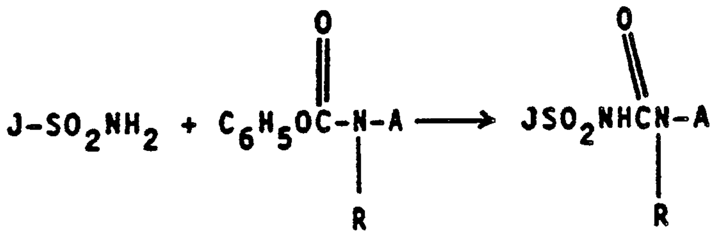

II XII I

wherein A and J are as defined in claim 7.

12. A herbicidal composition comprising a compound according to claim 1.

13. A process for severely damaging or killing unwanted plants which process comprises applying to the plants, or to the growth medium of the plants, an effective amount of a compound according to claim 1.

14. A process for controlling weeds in cultivated crops which process comprises applying to the crop, or to the growth medium of the crop, a compound according to claim 1 in an amount sufficient to severely damage or kill the weeds but insufficient to damage the crop substantially.

15. A process for regulating the growth of a plant which process comprises applying to the plant, to the seed of the plant, or to the growth medium of the plant, an effective amount of a compound according to claim 1.

16. A plant growth regulating composition comprising a compound according to claim 1 and an inert carrier therefor.

17. A herbicidal composition comprising a mixture of at least one compound according to claim 1 with at least one other herbicide.

18. A herbicidal composition according to claim 17, wherein the other herbicide has a complementary action to that of the compound according to claim 1.

19. A herbicidal composition according to claim 17, wherein the other herbicide is a contact herbicide.

European Patent Office

# EUROPEAN SEARCH REPORT

Application Number

EP 89 31 0040

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 099 339 (CIBA GEIGY AG)<br>* Page 27, ringsystem Q44; page 60, compounds 5.384-5.391; claims 1,21,22 *<br>& AU-A-1 688 893 (Cat. D)<br>--- | 1,9-12 | C 07 D 413/12<br>A 01 N 47/36 |
| A,D | EP-A-0 082 681 (E.I. DU PONT DE NEMOURS CO.)<br>* Pages 167-185; claims 1,31 *<br>--- | 1,9-12 | |
| E | EP-A-0 313 311 (ICI AUSTRALIA LTD)<br>* Comound 57; claims *<br>----- | 1-7,9-19 | |
| | | | TECHNICAL FIELDS SEARCHED (Int. Cl.5) |
| | | | C 07 D 413/00 |

The present search report has been drawn up for all claims

| Place of search | Date of completion of the search | Examiner |
|---|---|---|
| THE HAGUE | 27-10-1989 | DE JONG B.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document